(19) Europäisches Patentamt
European Patent Office
Office européen des brevets

(11) Veröffentlichungsnummer: **0 086 401**
**B2**

(12) # NEUE EUROPÄISCHE PATENTSCHRIFT

(45) Veröffentlichungstag der neuen Patentschrift: **18.10.89**

(51) Int. Cl.⁴: **C 07 C 69/675**, C 07 C 69/68, C 09 J 3/00

(21) Anmeldenummer: **83100985.7**

(22) Anmeldetag: **03.02.83**

(54) **Schwerflüchtige (Meth)-acrylate und ihre Verwendung.**

(30) Priorität: **10.02.82 DE 3204504**

(43) Veröffentlichungstag der Anmeldung: **24.08.83 Patentblatt 83/34**

(45) Bekanntmachung des Hinweises auf die Patenterteilung: **26.11.86 Patentblatt 86/48**

(45) Bekanntmachung des Hinweises auf die Entscheidung über den Einspruch: **18.10.89 Patentblatt 89/42**

(84) Benannte Vertragsstaaten: **AT BE CH DE FR GB IT LI NL SE**

(56) Entgegenhaltungen:
**EP-A- 0 003 045**
**DE-A- 2 020 419**
**DE-A- 2 103 870**
**US-A- 2 141 546**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Ritter, Wolfgang, Dr., Hochdahler Strasse 32, D-4010 Hilden (DE)**

EP 0 086 401 B2

**Beschreibung**

Innerhalb der Gruppe der Reaktivklebstoffe kommt den Methacrylatsystemen eine ständig steigende Bedeutung zu. In zweikomponentiger Formulierung sind sie seit Jahren im Markt eingeführt. Komponente A (Klebstoffkomponente) besteht üblicherweise aus der Lösung von Elastomeren in einem Monomeren beziehungsweise Monomerengemisch auf Basis von Acrylsäure und/oder insbesondere Methacrylsäure. Komponente B (Aktivator- oder Härterkomponente) enthält einen Initiator zur Auslösung radikalischer Polymerisationen.

Die beiden Komponenten werden vor der Anwendung intensiv vermischt und dadurch die Polymerisation der Monomeren eingeleitet.

Eine Weiterentwicklung dieser sogenannten 2-K-Systeme stellen die «No-Mix» oder «Acrylatklebstoffe der zweiten Generation» dar. Die Klebstoffanwendung gestaltet sich ähnlich wie bei den Kontaktklebstoffen: Zunächst wird die Initiatorkomponente in dünner Schicht auf eine oder beide der zu verklebenden Oberflächen aufgebracht. Nach einer Wartezeit, die bis zu Stunden betragen kann, wird die Klebstoffkomponente aufgetragen. Die Verklebung erfolgt durch Fixierung der Teile in der gewünschten Position.

In der Klebstoffkomponente werden als polymerisierbare Monomere insbesondere Methacrylsäure beziehungsweise Methacrylsäurederivate wie Methylmethacrylat, aber auch entsprechende Acrylsäureverbindungen, beispielsweise Butylacrylat, verwendet beziehungsweise mitverwendet. Neben diesen Monomeren wurden weitere Systeme untersucht, die teilweise eine gewisse technische Bedeutung gewonnen haben. Beispiele hierfür sind: Ethylenglycol- und Oligoethylen glycoldimethacrylate, (Meth)-acrylester von Tricyclo [5.2.1.0$^{2.6}$] decan, Glycerin(meth)acrylate, Bisphenol-A-Derivate, perfluorierte Dimethacrylate, aromatische Diester-Dimethacrylate, aromatische Diether- und aromatische Ester-Ether-Dimethacrylate, Urethan-Dimethacrylate, Diharnstoff-Dimethacrylate und andere Verbindungen. Aus der umfangreichen einschlägigen Literatur sei lediglich beispielhaft verwiesen auf: Polymer Science- and Technology 14, 357, 373 und 379 (1981); J. Dent. Res. 58, 1544 und 1981 (1979), J. Dent. Res. 58, Abstr. Nr. 1216, 395 (1979), J. Dent. Res. 49, 810 (1970) und 52, 731 sowie 1128 (1973), US-PSen 4 131 729, 3 066 112, 3 179 623, 3 810 938 sowie DE-OSen 2 432 013, 2 312 559 und 2 411 760.

Zur Kohäsionsverbesserung, zur Einstellung anwendungstechnisch günstiger Viskositäten und zur Verringerung der Volumenkontraktion, während des Härtens werden dem Monomeren in der Klebstoffkomponente Polymere zugemischt. Beispiele hierfür sind Polymethylmethacrylat, Polychloropren, chlorsulfoniertes Polyethylen, Nitrilkautschuke und/oder Polyurethane.

Die klassischen Härter für bei Raumtemperatur polymerisierende Methacrylatsysteme bestehen aus Benzoylperoxid als Initiator und tertiären aromatischen Aminen, insbesondere N,N-Dimethyltoluidin als Beschleuniger.

Heute steht eine breite Palette von Beschleunigern zur Verfügung, bedeutungsvoll sind dabei auch neue Härtersysteme auf Basis von Organo-Borverbindungen, die unter Zutritt von Luftsauerstoff befähigt sind, die Polymerisationsreaktion auszulösen, wobei insbesondere auch bei Raumtemperatur gearbeitet werden kann. Neue Härtersysteme auf Basis solcher bororganischer Verbindungen sind Gegenstand einer Reihe älterer Schutzrechtsanmeldungen der Anmelderin.

Reaktionsklebstoffe der geschilderen Art weisen eine Reihe von günstigen Eigenschaften auf. Sie besitzen beispielsweise ein breites Anwendungsspektrum auf unterschiedlichen Oberflächen bei geringem Aufwand der Oberflächenvorbehandlung, sie führen zu einer raschen Aushärtung gewünschtenfalls auch bei Raumtemperatur, durch geeignete Wahl von reaktiven Monomeren beziehungsweise Monomerengemischen können gute Flexibilitätswerte der Klebeschicht eingestellt werden. Es werden hohe Zugscherfestigkeiten erhalten. Bei der Klebstoffverarbeitung kann die sogenannte offene Zeit reguliert werden. Die Klebung erfolgt unter niedriger Schrumpfung und guter Reproduzierbarkeit unter Ausbildung einer Klebmasse, die gute Lösungsmittel- und Temperaturbeständigkeit besitzt.

Überwiegender Bestandteil der Klebstoffkomponente sind nach wie vor Mono(meth)-acrylate, die einen hohen Dampfdruck aufweisen. Hierdurch wird nicht nur eine Geruchsbelästigung bewirkt, es leitet sich auch daraus ab, dass die Klebstoffkomponente zum Beispiel bei No-Mix-Anwendungen nur kurz offen gelagert werden kann.

Darüber hinaus sind eine Reihe von Mono(meth)-acrylaten von Hydroxycarbonsäuren bzw. von deren Oligomeren aus dem US-Patent 2 141 546 und aus der europäischen Patentanmeldung 0 003 045 bekannt. Wenngleich diese Verbindungen überwiegend niederen Dampfdruck aufweisen, so sind sie doch für den Einsatzzweck als Reaktivklebstoffe wenig geeignet, da die mit diesen Monomeren durchgeführten Verklebungen nicht die geforderten hohen Zugscherfestigkeiten aufweisen.

Bismethacrylate besitzen üblicherweise höhere Siedepunkte. Viele dieser Typen – insbesondere Bismethacrylate mit hohem Molekulargewicht – sind jedoch fest und als Monomere in Klebstoffen nur in untergeordnetem Ausmass einzusetzen.

Bei Bismethacrylaten mit niedrigem Molekulargewicht ist der Abstand der funktionellen Gruppen gering und die entsprechenden Polymeren sind hoch vernetzt und neigen zum Verspröden.

Die vorliegende Erfindung geht von der Aufgabe aus, neue reaktive Monomere auf Acrylat- beziehungsweise Methacrylat-Basis – im folgenden als (Meth)-acrylatverbindungen bezeichnet – zu schaffen, die eine Mehrzahl wünschenswerter

Eigenschaften in der Anwendung und in den unter ihrer Mitverwendung hergestellten Verklebungen zeigen. Die neuen Monomeren sollen aufgrund ihres niedrigen Dampfdrucks eine geringe Flüchtigkeit zeigen. Trotz vergleichsweise hohem Molekulargewicht soll die Möglichkeit bestehen, sie bei Raumtemperatur als Flüssigkeiten oder wenigstens viskos-streichbare Pasten auszubilden. Wenn sie bei Raumtemperatur Feststoffe sind, sollen sie eine gute Mischbarkeit mit den üblicherweise eingesetzten weiteren Komponenten der hier betroffenen Klebstoffsysteme zeigen. Unter Verwendung der neuen Monomeren sollen sich hochfeste elastische Klebeverbindungen erzielen lassen.

Die technische Lösung der erfindungsgemässen Aufgabenstellung geht von der Feststellung aus, dass (Meth)-acrylatderivate bestimmter Polyester-Oligomere geeignet sind, die gewünschten technischen Effekte optimal zu kombinieren.

Gegenstand der Erfindung sind dementsprechend neue schwerflüchtige, bei Raumtemperatur flüssige bis feste (Meth)-acrylatverbindungen mit endständigen (Meth)-acrylsäureresten an einer Oligomeren-Kette, bei denen als Oligomeren-Kette ein aus Hydroxycarbonsäuren gebildetes Polyester-Oligomeres vorliegt, dadurch gekennzeichnet, dass sie zwei am Polyester-Oligomeren, die aus Glykolsäure und/oder Milchsäure aufgebaut sind, in alpha-omega-Stellung vorliegende (Meth)-acrylatreste aufweisen.

Die neuen (Meth)-acrylatverbindungen enthalten dabei zwei (Meth)-acrylatreste an dem Polyester-Oligomersegment. Die (Meth)-acrylatreste liegen endständig am Oligomersegment, so dass die beiden Endeinheiten (α, ω-Stellung) das Oligomersegments jeweils mit einer (Meth)-acrylatgruppe substituiert sind.

Die Oligomersegmente weisen das Strukturmerkmal

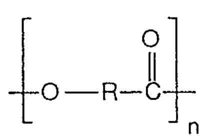

auf. Sie sind durch Oligomerisierung von Milchsäure und/oder Glycolsäure zugänglich. In einer bevorzugten Ausführungsform der Erfindung sind die Reste -P- und n derart ausgewählt beziehungsweise aufeinander abgestimmt, dass das mittlere Molekulargewicht der Polyester-Oligomereinheit im Bereich von etwa 200 bis etwa 600 liegt. Besonders bevorzugte Werte für das mittlere Molekulargewicht liegen im Bereich von etwa 300 bis 500.

Geeignete Hydroxycarbonsäuren zur Ausbildung dieses Mittelstücks der neuen (Meth)-acrylatverbindungen sind Glycolsäure und die isomeren Milchsäuren. Es können dabei bestimmte Isomere der genannten Säuren als auch Gemische zum Einsatz kommen.

Die Polyester-Oligomeren werden zweckmässigerweise unter Mitverwendung von difunktionellen Reaktanten hergestellt, die eine mehrfache Funktion erfüllen. Einerseits wird durch diese mitverwendeten Reaktanten die Regelung des mittleren Molekulargewichts im Polyester-Oligomeren und damit die Einstellung der angestrebten Viskositätsbereiche ermöglicht. Zum anderen ist durch Auswahl der funktionellen Gruppen der mitverwendeten Coreaktanten die Möglichkeit gegeben, dem Polyester-Oligomeren einheitlich endständige Hydroxylgruppen oder endständige Carboxylgruppen auszubilden, was an sich bei einem Polymeren beziehungsweise Oligomeren einer Hydroxycarbonsäure zunächst ja nicht der Fall ist.

Werden Oligomere der geschilderten Art durch Cokondensation von Hydroxycarbonsäuren und Diolen hergestellt, so entstehen letztlich Polyester-Oligomere mit endständigen Hydroxylgruppen. Die Menge der mitverwendeten Diole bestimmt – in Abstimmung mit den Reaktionsbedingungen – das mittlere Molekulargewicht des anfallenden Polyester-Oligomeren. Werden andererseits die Oligomeren durch Cokondensation von Hydroxycarbonsäuren mit Dicarbonsäuren beziehungsweise reaktiven Dicarbonsäurenderivaten hergestellt, so entstehen Polyester-Oligomere mit endständigen Carboxylgruppen beziehungsweise Carboxylgruppenderivate. Auch hier wirkt der mitverwendete Coreaktant gleichzeitig vereinheitlichend bezüglich der endständigen reaktiven Gruppen und molekulargewichtsregelnd. Im einzelnen gilt hier das bekannte Wissen zur Herstellung von Polyester beziehungsweise Copolyestern.

In allen hier geschilderten Fällen entstehen modifizierte Polyester-Oligomere, die in an sich bekannter Weise einfach zu (Meth)-acrylatverbindungen umgesetzt werden können. Liegen endständige Hydroxylgruppen am Polyester-Oligomeren vor, so wird die (Meth)-acrylsäuregruppe durch Veresterung beziehungsweise Umesterung – oder aber durch vergleichbare Reaktion mit Acrylsäure beziehungsweise Acrylsäureestern und/oder insbesondere entsprechenden Methacrylsäureverbindungen eingeführt. Liegen im Polyester-Oligomeren jedoch endständige Carboxylgruppen vor, so lassen sich ebenfalls in an sich bekannter Weise leicht die gewünschten (Meth)-acrylatgruppen anbinden. Geeignet ist hier beispielsweise die Umsetzung des oligomeren Zwischenproduktes mit Glycidylestern der Acrylsäure beziehungsweise der Methacrylsäure. Unter Aufspaltung der Glycidylgruppierung wird die (Meth)acrylatgruppe über den Glyceridrest an die intermediär gebildete Mono- beziehungsweise Dicarbonsäure angebunden.

In der Auswahl der in der Regel nur in untergeordneten Mengen mitverwendeten difunktionellen Coreaktanten sind praktisch keine Einschränkungen gegeben. So können diese Reaktanten aliphatischer, cycloaliphatischer oder aromatischer Natur sein. Sie besitzen im allgemeinen eine Kohlenstoffzahl nicht über $C_{25}$, vorzugsweise nicht über $C_{15}$. Geeignete Diole enthalten beispielsweise 2 bis 20 C-Atome, vorzugsweise 2

bis 10 und insbesondere 2 bis 6 C-Atome im Molekül. Die gleichen Angaben gelten für die entsprechenden Dicarbonsäuren. Beispiele für geeignete Diole sind: Ethylenglycol, Di-, Tri-, Tetraethylenglycol, Propylenglycol, Dipropylenglycol, 1,3-Butylenglycol, 1,4-Butylenglycol, 2,2-Dimethyl-1,3-propandiol, 2,2,4-Trimethyl-1,6-hexandiol, 1,4-Cyclohexandimethanol, 1,5-Pentandiol, 1,6-Hexandiol, 1,10-Decandiol und 2,2-Bis-(4-hydroxy-cyclohexyl)-propan.

Geeignete Dicarbonsäuren sind beispielsweise: Oxalsäure, Malonsäure, Bernsteinsäure, Glutarsäure, Adipinsäure, Pimelinsäure, Korksäure, Azelainsäure, Sebazinsäure, Isosebazinsäure, Nonandicarbonsäure, Decandicarbonsäure, Undecandicarbonsäure, Dodecandicarbonsäure, Phthalsäure, Hexahydrophthalsäure, Isophthalsäure, Terephtalsäure, und Biphenyldicarbonsäure.

Als Säureanhydride eignen sich beispielsweise: Bernsteinsäureanhydrid, Glutarsäureanhydrid, Phthalsäureanhydrid, Itaconsäureanhydrid, Citraconsäureanhydrid ebenso wie Alkenylbernsteinsäureanhydride.

Selbstverständlich können in allen Fällen – das heisst sowohl bei der Hydroxycarbonsäuren als auch bei den Coreaktanten – nicht nur die jeweils freien reaktiven Komponenten der genannten Art, sondern auch solche reaktive Derivate eingesetzt werden, die in an sich bekannter Weise unter den Bedingungen der Veresterung beziehungsweise Umesterung die gewünschten Polyester-Oligomeren des vorbestimmten Molekulargewichts bilden. Geeignet sind also beispielsweise die Ester der Hydroxycarbonsäuren sowie Lactone beziehungsweise Lactame von Hydroxycarbonsäuren, die mit Diolen beziehungsweise Diolestern umgesetzt, insbesondere umgeestert werden können. Sowohl die Herstellung der Polyester-Oligomeren als auch deren Umsetzung zu den (Meth)-acrylatverbindungen erfolgt in an sich bekannter Weise zum Beispiel durch Reaktion in Gegenwart oder in Abwesenheit von Lösungsmitteln, gewünschtenfalls in Gegenwart von Katalysatoren, insbesondere Veresterungskatalysatoren.

Die neuen (Meth)-acrylatkomponenten sind wertvolle Bestandteile in Reaktionsklebstoffen in an sich bekannter Zusammensetzung. So können sie in 2-Komponenten-Reaktions-Klebstoffen der eingangs geschilderten Art mit Vorteil verwendet werden. Sie können dabei sowohl in konventionellen (Meth)-acrylatsystemen als in No-Mix-Klebstoffsystemen Verwendung finden. Sie sind geeignet, in den sogenannten Konstruktionsklebstoffen zum Verkleben von Metallen, Holz, Glas, Keramik und/oder Kunststoffen Verwendung zu finden.

In einer bevorzugten Ausführungsform der Erfindung sind die neuen (Meth)-acrylatverbindungen bei Raumtemperatur fliessfähig oder wenigstens noch pastös verstreichbar, so dass sie als Hauptkomponente oder sogar als alleinige polymerisierbare Klebstoffkomponente in den Reaktionsklebstoffen eingesetzt werden können. Es kann dabei bevorzugt sein, das die flüssigen (Meth)-acrylatverbindungen der Erfindung bei Raumtemperatur eine Viskosität im Bereich von 2000 bis 70000 mPas, vorzugsweise im Bereich von 3000 bis 50000 mPas, besitzen. Feste (Meth)-acrylatverbindungen der erfindungsgemässen Art lösen sich jedoch gut in flüssigen polymerisierbaren Komponenten, beispielsweise in Methylmethacrylat, so dass also auch solche festen Verbindungen der Erfindung in Abmischung mit flüssigen konventionellen Monomerbestandteilen in einfacher Weise zu den verarbeitungstechnisch gewünschten, flüssigen Klebstoffkomponenten aufgearbeitet werden können.

In den folgenden Beispielen wird zunächst die Herstellung verschiedener Typen von Polyester-Oligomeren (Beispiele 1 bis 10), dann die Herstellung der (Meth)-acrylat-Derivate dieser Polyesteroligomeren (Beispiele 11 bis 20) und schliesslich die Austestung dieser reaktiven Monomeren in Konstruktionsklebstoffen geschildert.

Beispiele 1–6

Oligohydroxycarbonsäuren mit Hydroxylendgruppen

a) Darstellung aus Glycolsäure und Ethylenglycol

In einem Dreihalskolben mit Rührer und Destillationsbrücke werden Glycolsäure und Ethylenglycol vorgelegt. Unter Stickstoff wird schnell auf 150 °C und dann im Verlauf von 6 Stunden von 150 auf 200 °C hochgeheizt. Dabei spaltet sich bereits der grösste Anteil des Reaktionswassers, der den Umsatz der Esterkondensation anzeigt, ab. Man lässt den Ansatz auf etwa 150 °C abkühlen, evakuiert vorsichtig auf 10 Torr und vervollständigt den Umsatz bei 200 °C und 10 Torr. Nach 30 Minuten wird das Produkt bei ca. 150 °C heiss abgefüllt. Die Zusammensetzung der Ansätze und die Oligomereigenschaften sind der Tabelle 1 (Beispiele 1–3) zu entnehmen.

Tabelle 1

Oligohydroxycarbonsäuren mit Hydroxylendgruppen aus Glycolsäure und Ethylenglycol

| Beispiel | Edukte<br>Glycolsäure<br>mol | <br>Ethylenglycol<br>mol | Reaktionswasser<br>% d. Th. | Säurezahl | Berechnetes Molgewicht<br>g/mol | Beschaffenheit |
|---|---|---|---|---|---|---|
| 1 | 3 | 1 | 100 | 14 | 252 | klar, viskos, hellgelb |
| 2 | 4 | 1 | > 90 | 20 | 294 | viskos, weiss |
| 3 | 6 | 1 | > 98 | 24 | 410 | wachsartig, weiss |

b) Darstellung aus Milchsäureethylester und Ethylenglycol

In einem Dreihalskolben mit Rührer und Destillationsbrücke werden Milchsäureethylester und Ethylenglycol vereinigt. Als Katalysator werden 70 ml 0,2%ige methanolische Natriummethylatlösung zugegeben und der Ansatz im Anschluss auf 150°C erhitzt. Man steigert die Temperatur vorsichtig weiter bis auf 180°C, so dass bei ca. 80°C konstant Ethanol abdestilliert. Nach dem Ende der Ethanolabspaltung nach ca. 16 Stunden wird dabei 150°C Badtemperatur auf 10 Torr evakuiert und die Substanz im Anschluss unter Stickstoff abgefüllt. Die Zusammensetzung der Ansätze und die Oligomereigenschaften sind der Tabelle 2 (Beispiele 4–6) zu entnehmen.

Tabelle 2

Oligohydroxycarbonsäuren mit Hydroxylendgruppen aus Milchsäureethylester und Ethylenglycol

| Beispiel | Edukte | | Produkte | | Beschaffenheit |
|---|---|---|---|---|---|
| | Milchsäureethylester mol | Ethylenglycol mol | Ausbeute Ethanol, % | Molgewicht | |
| 4 | 2 | 1 | 95 | 193 (Osmose) | klar, viskos, orangenfarben |
| 5 | 4 | 1 | 92 | | klar, viskos, braun |
| 6 | 6 | 1 | 96 | | klar, viskos, rotbraun |

Beispiele 7–10

Oligohydroxycarbonsäuren mit Carboxylendgruppen

Allgemeine Vorschrift

a) Durch Umsetzung von hydroxylgruppenterminierten Oligohydroxycarbonsäuren mit Bernsteinsäureanhydrid, Beispiel 7

206,3 g des Oligomeren aus Beispiel 4, 200 g Bernsteinsäureanhydrid und 1 g Benzyltrimethylymmoniummethoxid (40%ig in Methanol) werden in einem Dreihalskolben mit Rührer, Rückflusskühler und Stickstoffeinleitung vereinigt und 8 Stunden bei 80°C unter Stickstoff gerührt. Das Produkt ist durch folgende Kennzahlen charakterisiert: Säurezahl: 296, OH-Zahl: $<3$.

b) Darstellung aus Glycolsäure und Adipinsäure

In einem Dreihalskolben mit Rührer und Destillationsbrücke werden Dicarbonsäure und Hydroxycarbonsäure vorgelegt. Unter Stickstoff wird schnell auf 150°C und dann im Verlauf von 6 Stunden von 150 auf 200°C hochgeheizt. Dabei spaltet sich bereits der grösste Teil des Reaktionswassers, der den Umsatz der Esterkondensation anzeigt, ab. Man lässt den Ansatz auf etwa 150°C abkühlen, evakuiert vorsichtig auf 10 Torr und vervollständigt den Umsatz bei 200°C und 10 Torr. Das Produkt wird heiss unter Stickstoff abgefüllt. Die Zusammensetzung der Ansätze und die Oligomereigenschaften sind der Tabelle 3 (Beipsiele 8 bis 10) zu entnehmen.

Tabelle 3

Oligohydroxycarbonsäuren mit Carboxylendgruppen aus Glycolsäure und Adipinsäure

| Beispiel | Edukte | | Produkte | | | Beschaffenheit |
|---|---|---|---|---|---|---|
| | Glycolsäure mol | Adipinsäure mol | Ausbeute Reaktions-wasser, % | Säurezahl | Molgewicht aus Säurezahl g $mol^{-1}$ | |
| 8 | 1 | 1 | $>99$ | 620 | 181 | wachsartig, fest |
| 9 | 3 | 1 | $>98$ | 376 | 298 | wachsartig, weich |
| 10 | 4 | 1 | $>96$ | 334 | 336 | wachsartig, weich |

Beispiele 11–20

Oligohydroxycarbonsäuren mit polymerisierbaren Endgruppen

a) Oligohydroxycarbonsäuren mit polymerisierbaren Endgruppen aus Oligohydroxycarbonsäuren mit endständigen Hydroxylgruppen

In einem Dreihalskolben mit Rührer und Wasserabscheider werden die Oligohydroxycarbonsäuren mit Hydroxylendgruppen und 1,1 Äquivalent Methacrylsäure pro Hydrocxylgruppe vorgelegt. Gleichzeitig werden – bezogen auf Methacrylsäure – gleiche Gewichtsteile Toluol und jeweils 2 Gewichtsprozent p-Toluolsulfonsäure und Hydrochinon zugegeben.

Das Gemisch wird unter schnellem Rühren zum Sieden erhitzt und das gebildete Reaktionswasser am Wasserabscheider abgetrennt.

Wenn sich nach 5 Stunden Reaktionszeit weniger als 90 bis 95 Gewichtsprozent des theoretisch zu erwartenden Reaktionswasser abgespaltet haben, werden nochmals 20% der ursprünglich zugegebenen Menge Methacrylsäure mit jeweils 2 Gewichtsprozent para-Toluolsulfonsäure und Hydrochinon zugegeben und die Reaktion weitergeführt.

Die Reaktion is beendet, sobald sich 90 bis 95% des zu erwartenten Reaktionswassers gebildet haben. Das Reaktionsprodukt wird nach dem Erkalten in das doppelte Volumen Ethanol gegeben und filtriert. Die klare ethanolische Lösung engt man am Rotationsverdampfer auf ¼ des ursprünglichen Volumens ein, giesst sie in die gleiche Menge Wasser und neutralisiert sie mit Natriumhydrogencarbonat. Die organische Phase wird abgetrennt, die wässrige Phase mit Toluol ausgeschüttelt, die organischen Phasen vereinigt, dreimal mit Wasser gewaschen und über Natriumsulfat getrocknet. Das Lösungsmittel wird bei Raumtemperatur zunächst am Rotationsverdampfer, dann bei $10^{-4}$ Torr abgezogen. Die Zusammensetzung der Ansätze und die Eigenschaften der polymerisierbaren Oligomeren sind in der Tabelle 4 (Beispiele 11–16) aufgeführt.

Tabelle 4

Oligohydroxycarbonsäuren mit polymerisierbaren Endgruppen

| Beispiel | Edukt Diol aus Beispiel | Ausbeute Reaktionswasser, % | Beschaffenheit |
|---|---|---|---|
| 11 | 1 | >97 | homogen, dünnflüssig, braun |
| 12 | 2 | >95 | homogen, dünnflüssig, braun |
| 13 | 3 | >95 | homogen, dünnflüssig, braun |
| 14 | 4 | >95 | homogen, dünnflüssig, braun |
| 15 | 5 | >95 | homogen, viskos, braun |
| 16 | 6 | >90 | homogen, viskos, braun |

b) Oligohydroxycarbonsäuren mit polymerisierbaren Endgruppen aus Oligohydroxycarbonsäuren mit endständigen Carboxylgruppen

In einem Dreihalskolben mit Rührer und Rückflusskühler wird die Oligohydroxycarbonsäure mit endständigen Carboxylgruppen vorgelegt und 1,0 Äquivalent Glycidylmethacrylat mit 0,1 Gewichtsprozent Benzyltrimethylammoniummethoxid und 0,06 Gewichtsprozent Hydrochinon zugegeben. Unter Rühren wird das Gemisch innerhalb von 45 Minuten auf 80°C erhitzt und die Reaktion bei 80°C so lange weitergeführt, bis die Säurezahl unter 35 abgesunken ist. Die Reaktion ist üblicherweise nach 10 bis 20 Stunden beendet.

Die Zusammensetzung der Ansätze und die Eigenschaften der polymerisierbaren Oligomeren sind in der Tabelle 5 (Beispiele 17–20) aufgeführt.

Tabelle 5

Oligohydroxycarbonsäuren mit polymerisierbaren Endgruppen

| Beispiel | Edukt Dicarbonsäure aus Beispiel | Produkt Säurezahl | Beschaffenheit |
|---|---|---|---|
| 17 | 7 | 24 | homogen, viskos, gelb |
| 18 | 8 | 30 | homogen, viskos, hellgelb |
| 19 | 9 | 34 | homogen, hochviskos, hellgelb |
| 20 | 10 | 31 | homogen, hochviskos, hellgelb |

Beispiel 21

Konstruktive Verklebungen mit Oligohydroxycarbonsäuren mit polymerisierbaren Endgruppen

Mit den dargestellten Monomeren wurden an sandgestrahlten und entfetteten Eisenblechen (DIN 53 281/53 283) Verklebungen durchgeführt.

Dazu wurden die dargestellten Oligohydroxycarbonsäuren mit polymerisierbaren Endgruppen I. in reiner Form, II. in einer Mischung mit 20 Gewichtsprozent Hydroxyethylmethacrylat, III. in einer Mischung mit 20 Gewichtsprozent Methylmethacrylat und 5 Gewichtsprozent Methacrylsäure eingesetzt.

a) Zu jeweils 5 g der Monomeren (I) beziehungsweise der Monomerengemische (II, III) werden unter intensivem Mischen zunächst 0,5 Gewichtsprozent N,N-Dimethyl-p-toluidin und im Anschluss 0,5 Gewichtsprozent Dibenzoylperoxid zugegeben. Die Mischungen haben Topfzeiten zwischen 2 und 8 Minuten. Mit dem Klebstoff wurden sandgestrahlte und entfettete Eisenbleche verklebt und nach 24stündiger Lagerung bei Raumtemperatur an der Luft die Zugscherfestigkeiten bestimmt.

b) In einer weiteren Versuchsreihe wurden jeweils 5 g der Monomeren (I) beziehungsweise der

Monomerengemische (II, III) mit 1 Gewichtsprozent Dibenzoylperoxid versetzt, jeweils weitere 5 g Monomer mit 1 Gewichtsprozent N,N-Dimethyl-p-toluidin. Ein Teil der Eisenprüfkörper wurde mit dem peroxid-, der andere mit dem dimethyltoluidinhaltigen Monomer bestrichen.

Unterschiedlich beschichtete Prüfkörper wurden paarweise zusammengefügt und fixiert. Die Prüfkörper wurden 24 h bei Raumtemperatur gelagert und dann im Zugscherversuch zerrissen. Die gemessenen Zugscherfestigkeiten sind in der Tabelle 6 aufgelistet. Die angegebenen Zugscherfestigkeiten sind arithmetische Mittelwerte aus jeweils 6 Verklebungen nach a) und b) bei einer Zerreissgeschwindigkeit von 12 mm/Minute.

Tabelle 6

Zugscherfestigkeiten (ZSF) bei der Verklebung von Eisenblechen mit Monomerklebstoffen auf Basis von Oligohydroxycarbonsäuren mit polymerisierbaren Endgruppen/$Nmm^{-2}$

| Monomer aus Beispiel | Comonomere I – | II Hydroxyethylmethacrylat 20 Gew.-% | III Methylmethacrylat, 20 Gew.-% Methacrylsäure, 5 Gew.-% |
|---|---|---|---|
| 11 | 15 | 16 | 18 |
| 12 | 17 | 20 | 18 |
| 13 | 18 | 18 | 19 |
| 14 | 13 | 15 | 16 |
| 15 | 16 | 16 | 17 |
| 16 | 8 | 16 | 16 |
| 17 | 11 | 18 | 20 |
| 18 | 17 | 21 | 22 |
| 19 | 7 | 24 | 23 |
| 20 | 6 | 21 | 19 |

**Patentansprüche für die Vertragsstaaten: BE, CH, DE, FR, GB, IT, LI, NL, SE**

1. Neue schwerflüchtige, bei Raumtemperatur flüssige bis feste (Meth)-acrylatverbindungen mit endständigen (Meth)-acrylsäureresten an einer Oligomeren-Kette, bei denen als Oligomeren-Kette ein aus Hydroxycarbonsäuren gebildetes Polyester-Oligomeres vorliegt, dadurch gekennzeichnet, dass sie zwei am Polyester-Oligomeren, die aus Glykolsäure und/oder Milchsäure aufgebaut sind, in alpha-omega-Stellung vorliegende (Meth)-acrylatreste aufweisen.

2. (Meth)-acrylatverbindungen nach Anspruch 1, dadurch gekennzeichnet, dass das Polyester-Oligomere ein mittleres Molekulargewicht im Bereich von 200 bis 600, vorzugsweise im Bereich von 300 bis 500, hat.

3. (Meth)-acrylatverbindungen nach den Ansprüchen 1 und 2, dadurch gekennzeichnet, dass die Polyester-Oligomeren unter Mitverwendung von difunktionellen Alkoholen, Carbonsäuren bzw. Carbonsäureanhydriden hergestellt worden sind.

4. (Meth)-acrylatverbindungen nach den Ansprüchen 1 bis 3, dadurch gekennzeichnet, dass sie bei Raumtemperatur fliessfähig bis pastös verstreichbar sind und dabei bei Raumtemperatur eine Viskosität im Bereich von 2000 bis 70000 mPas aufweisen.

5. Verwendung der (Meth)-acrylatverbindungen nach den Ansprüchen 1 bis 4 als reaktive Monomerkomponente in Reaktionsklebstoffen zum Verbinden von Metall, Holz, Glas, Keramik und/oder Kunststoffen.

**Patentansprüche für: AT**

1. Verfahren zur Herstellung schwerflüchtiger, bei Raumtemperatur flüssiger bis fester (Meth)-acrylatverbindungen mit endständigen (Meth)-acrylsäureresten an einer Oligomeren-Kette, bei denen als Oligomeren-Kette ein aus Hydroxycarbonsäuren gebildetes Polyester-Oligomeres vorliegt, dadurch gekennzeichnet, dass sie zwei am Polyester-Oligomeren, das aus Glykolsäure und/oder Milchsäure aufgebaut ist, in alpha-omega-Stellung vorliegende (Meth)-acrylatreste aufweisen.

2. Verfahren nach Anspruch 1, dadurch gekennzeichnet, dass das Polyester-Oligomere ein mittleres Molekulargewicht im Bereich von 200 bis 600, vorzugsweise im Bereich von 300 bis 500, hat.

3. Verfahren den Ansprüchen 1 und 2, dadurch gekennzeichnet, dass die Polyester-Oligomeren unter Mitverwendung von difunktionellen Alkoholen, Carbonsäuren bzw. Carbonsäureanhydriden hergestellt worden sind.

4. Verfahren nach den Ansprüchen 1 bis 3, dadurch gekennzeichnet, dass sie bei Raumtemperatur fliessfähig bis pastös verstreichbar sind und dabei bei Raumtemperatur eine Viskosität im Bereich von 2000 bis 70000 mPas aufweisen.

5. Verwendung der (Meth)-acrylatverbindungen nach den Ansprüchen 1 bis 4 als reaktive Monomerkomponente in Reaktionsklebstoffen zum Verbinden von Metall, Holz, Glas, Keramik und/oder Kunststoffen.

**Claims for the contracting states: BE, CH, DE, FR, GB, IT, LI, NL, SE**

1. New, low-volatility (meth)acrylate compounds liquid to solid at room temperature and terminated by (meth)acrylic acid groups on an oligomer chain, in which a polyester oligomer formed from hydroxycarboxylic acids is present as the oligomer chain, characterized in that they comprise two (meth)acrylate groups in the α, ω-position on the polyester oligomer which is synthesized from glycolic acid and/or lactic acid.

2. (Meth)acrylate compounds as claimed in claim 1, characterized in that the polyester oligomer has an average molecular weight in the range from 200 to 600 and preferably in the range from 300 to 500.

3. (Meth)acrylate compounds as claimed in claims 1 and 2, characterized in that the polyester oligomers were prepared using difunctional alcohols, carboxylic acids or carboxylic anhydrides.

4. (Meth)acrylate compounds as claimed in claims 1 to 3, characterized in that they are fluid to paste-like and spreadable at room temperature with a viscosity at room temperature in the range from 2,000 to 70,000 mPa.s.

5. The use of the (meth)acrylate compounds claimed in claims 1 to 4 as reactive monomer component in reaction adhesives for bonding metals, wood, glass, ceramics and/or plastics.

**Claims for the contracting State: AT**

1. A process for the production of low-volatility (meth)-acrylate compounds liquid to solid at room temperature and terminated by (meth)acrylic acid groups on an oligomer chain, in which a polyester oligomer formed from hydroxycarboxylic acids is present as the oligomer chain, characterized in that they comprise two (meth)acrylate groups in the α, ω-position on the polyester oligomer which is synthesized from glycolic acid and/or lactic acid.

2. A process as claimed in claim 1, characterized in that the polyester oligomer has an average molecular weight in the range from 200 to 600 and preferably in the range from 300 to 500.

3. A process as claimed in claims 1 and 2, characterized in that the polyester oligomers were prepared using difunctional alcohols, carboxylic acids or carboxylic anhydrides.

4. A process as claimed in claims 1 to 3, characterized in that they are fluid to paste-like and spreadable at room temperature with a viscosity at room temperature in the range from 2,000 to 70,000 mPa.s.

5. The use of the (meth)acrylate compounds claimed in claims 1 to 4 as reactive monomer component in reaction adhesives for bonding metals, wood, glass, ceramics and/or plastics.

**Revendications (pour les états contractants: BE, CH, DE, FR, GB, IT, LI, NL, SE**

1. Nouveaux composés (meth)acryliques difficilement volatils, liquides à solides à la température ambiante et avec des radicaux (méth)acryliques terminaux sur une chaîne oligomère, dans lesquels comme chaîne oligomère on a un polyester oligomère formé au départ d'acides hydroxycarboxyliques, caractérisés en ce qu'ils présentent deux radicaux (méth)acryliques se trouvant en position α, ω sur le polyester oligomère, formé d'acide glycolique et/ou d'acide lactique.

2. Composés (méth)acryliques selon la revendication 1, caractérisés en ce que le polyester oligomère possède un poids moléculaire moyen dans l'intervalle de 200 à 600, de préférence dans l'intervalle de 300 à 500.

3. Composés (méth)acryliques selon les revendications 1 et 2, caractérisés en ce que les polyesters oligomères sont préparés par l'emploi conjoint d'alcools, d'acides carboxyliques ou d'anhydrides d'acides carboxyliques difonctionnels.

4. Composés (méth)acryliques selon les revendications 1 à 3, caractérisés en ce qu'ils sont étendables à la température ambiante à l'état fluide à pâteux et présentent à la température ambiante une viscosité dans l'intervalle de 2000 à 70.000 mPas.

5. Utilisation des composés (méth)acryliques selon les revendications 1 à 4 comme composant monomère réactif dans des adhésifs réactifs pour le collage du métal, du bois, du verre, de la céramique et/ou des matières synthétiques.

**Revendications pour l'etat contractant: AT**

1. Procédé de fabrication de composés (méth)acryliques difficilement volatils, liquides à solides à la température ambiante et avec des radicaux (méth)acryliques terminaux sur une chaîne oligomère, comportant comme chaîne oligomère un polyester oligomère formé d'acides hydroxycarboxyliques, caractérisé en ce qu'ils présentent deux radicaux (méth)acryliques en position α, ω du polyester oligomère formé à partir d'acide glycolique et/ou d'acide lactique.

2. Procédé selon la revendication 1, caractérisé en ce que le polyester oligomère a un poids moléculaire moyen dans l'intervalle de 200 à 600, de préférence dans l'intervalle de 300 à 500.

3. Procédé selon les revendications 1 et 2, caractérisé en ce que les polyesters oligomères ont été préparés avec utilisation conjointe d'alcools, d'acides carboxyliques ou d'anhydrides d'acides carboxyliques difonctionnels.

4. Procédé selon les revendications 1 à 3, caractérisé en ce qu'ils sont étendables à la température ambiante à l'état fluide à pâteux et en ce qu'à la température ambiante ils présentent une viscosité dans l'intervalle de 2000 à 70.000 mPas.

5. Utilisation des composés (méth)acryliques préparés selon les revendications 1 à 4 comme composant monomère réactif dans des adhésifs réactifs pour le collage du métal, du bois, du verre, de la céramique et/ou des matières synthétiques.